(19) Europäisches Patentamt
European Patent Office
Office européen des brevets

(11) Numéro de publication: **0 193 432**
**A1**

(12) # DEMANDE DE BREVET EUROPEEN

(21) Numéro de dépôt: **86400207.6**

(22) Date de dépôt: **31.01.86**

(51) Int. Cl.⁴: **C 07 D 303/36**
A 61 K 31/335, C 07 C 91/15

(30) Priorité: **01.02.85 FR 8501426**

(43) Date de publication de la demande:
**03.09.86 Bulletin 86/36**

(84) Etats contractants désignés:
**AT BE CH DE FR GB IT LI LU NL SE**

(71) Demandeur: **CENTRE NATIONAL DE LA RECHERCHE SCIENTIFIQUE (CNRS)**
**15, Quai Anatole France**
**F-75007 Paris(FR)**

(71) Demandeur: **INSTITUT NATIONAL DE LA SANTE ET DE LA RECHERCHE MEDICALE (INSERM)**
**101, rue de Tolbiac**
**F-75013 Paris(FR)**

(72) Inventeur: **Doutheau, Alain**
**8, Rue Malesherbes**
**F-69006 Lyon(FR)**

(72) Inventeur: **Gore, Jacques**
**7, rue du Mont Cindre**
**F-69300 Caluire(FR)**

(72) Inventeur: **Quash, Gérard**
**Village de l'Ouest 12, Allée des Charmilles**
**F-69340 Francheville(FR)**

(72) Inventeur: **Bernard, Didier**
**1, rue Marietton**
**F-69009 Lyon(FR)**

(74) Mandataire: **Nony, Michel**
**29, rue Cambacérès**
**F-75008 Paris(FR)**

(54) **Nouveaux dérivés époxydiques, leur préparation et leur application, notamment comme médicaments antitumoraux.**

(57) Composés de formule I:

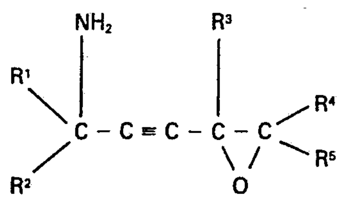

(I) ,

dans laquelle $R^1$ et $R^2$ représentent chacun indépendamment un groupement hydrocarboné pouvant être substitué et/ou comporter des hétéroatomes, ou bien $R^1$ et $R^2$ représentent ensemble un groupement hydrocarboné éventuellement substitué formant, avec l'atome de carbone auxquels ils sont attachés, un cycle pouvant comporter des hétéroatomes,

$R^3$ représente –H ou un groupement alkyle inférieur ou aryle, et

$R^4$ et $R^5$ représentent chacun indépendamment, –H ou un groupement hydrocarboné ; ainsi que leurs sels d'addition ; leur préparation et leur application, notamment comme médicaments antitumoraux.

Croydon Printing Company Ltd.

"Nouveaux dérivés époxydiques, leur préparation et leur application, notamment comme médicaments antitumoraux".

La présente invention a pour objet de nouveaux dérivés époxydiques possédant une activité d'inhibiteurs d'enzyme, leur préparation et leur application notamment comme médicaments antitumoraux.

On sait que l'aldehyde réductase (ALDR) est une des enzymes qui interviennent dans le processus de production de l'aldéhyde malonique à partir des polyamines en transformant cet aldéhyde en 1,3-propanediol et en l'empêchant ainsi de s'accumuler dans le milieu cellulaire.

On a maintenant découvert de nouveaux dérivés époxydiques qui sont des inhibiteurs d'ALDR et qui sont capables d'inhiber la croissance des cellules transformées.

La présente invention a pour objet les composés de formule générale I :

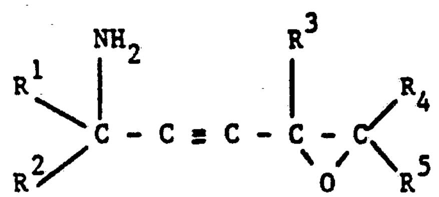

(I)

dans laquelle $R^1$ et $R^2$ représentent chacun indépendamment un groupement hydrocarboné pouvant être substitué et/ou comporter des hétéroatomes, ou bien $R^1$ et $R^2$ représentent ensemble un groupement hydrocarboné éventuellement substitué formant, avec l'atome de carbone auxquels ils sont attachés, un cycle pouvant comporter des hétéroatomes,

$R^3$ représente -H ou un groupement alkyle inférieur ou aryle,

et $R^4$ et $R^5$ représentent chacun indépendamment -H ou un groupement hydrocarboné.

Parmi les composés de formule I, on citera notamment :

- ceux pour lesquels $R^1$ et $R^2$ représentent chacun indépendamment un groupement alkyle ayant 1 à 5 atomes de carbone ; ou $R^1$ et $R^2$ représentent ensemble un groupement pentaméthylène ;

- ceux pour lesquels $R^3$ représente un groupement alkyle ayant 1 à 5 atomes de carbone ou un groupement phényle ;

- ceux pour lesquels $R^4$ et $R^5$ représentent chacun -H ;

- ou bien ceux pour lesquels l'un des substituants $R_4$, $R_5$ représente -H et l'autre représente un alkyle ayant 1 à 5 atomes de carbone.

L'invention concerne également les sels d'addition des composés de formule I. Les sels d'addition des composés de formule I sont notamment ceux formés avec les acides compatibles avec une utilisation en thérapeutique.

L'invention a également pour objet le procédé de préparation des composés de formule I.

Ce procédé est caractérisé par le fait que l'on utilise comme produit de départ un composé de formule II :

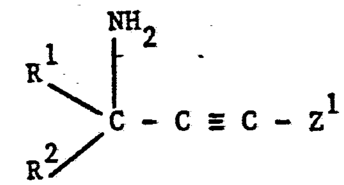

(II)

dans laquelle $Z^1$ représente soit -H soit un équivalent d'un métal ou d'un groupement lié à l'atome de carbone auquel il est attaché par un liaison carbone-métal, que l'on fait réagir ledit composé de départ avec un dérivé carbonylé alpha-halogéné de formule III :

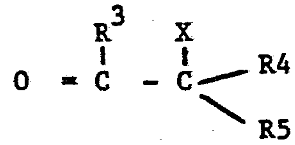

(III)

dans laquelle $R^3$, $R^4$ et $R^5$ sont définis comme précédemment et X représente un atome d'halogène,
pour obtenir une halohydrine de formule IV

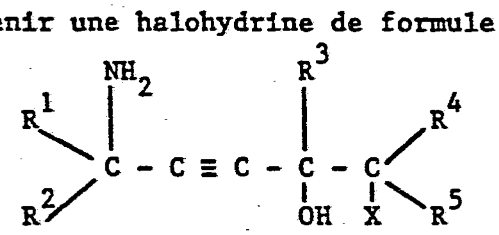

(IV)

dans laquelle $R^1$, $R^2$, $R^3$, $R^4$, $R^5$ et X sont définis comme précédemment, et que l'on soumet ledit composé de formule IV à l'action d'une base pour obtenir un composé de formule I, puis que l'on soumet le cas échéant le composé obtenu à une réaction de salification du groupement amine.

Les produits de départ de formule II sont connus ou peuvent être préparés selon les méthodes connues, par exemple selon la méthode décrite par G.F. HENNION et E.G. TEACH, J. ORG. CHEM. 1972, 17, 1653.

Les dérivés de formule III peuvent être préparés selon les méthodes générales de préparation des dérivés alpha-halo carbonylés.

Le procédé de l'invention peut encore présenter les caractéristiques suivantes, prises isolément ou en combinaison :

- Les dérivés organométalliques de formule II sont notamment ceux pour lesquels $Z^1$ représente un métal alcalin ou un groupement -MgHal, Hal étant un halogène ; par exemple avant réaction avec le dérivé carbonylé de formule III, on transforme au préalable un composé de formule II (avec $Z^1$=-H) en dérivé métallique correspondant (notamment organolithien ou organomagnésien) selon les méthodes usuelles ; on peut faire réagir en particulier le composé de formule II ($Z^1$=-H) avec un alkyl lithium pour former le dérivé lithié correspondant de formule II ($Z^1$=Li) ; ou bien on prépare, selon les méthodes usuelles, un dérivé organomagnésien correspondant ;

- pour transformer l'halohydrine IV en composé de formule I, on opère dans les conditions classiques de synthèse des époxydes au départ des halohydrines c'est-à-dire en présence d'une base telle qu'un alcoolate alcalin, par exemple un tert-butylate alcalin.

Les composés de formule I peuvent être transformés en tout sel d'addition correspondant par l'action d'un acide, par exemple un acide halohydrique.

L'invention a également pour objet, à titre de produits intermédiaires obtenus lors de la mise en oeuvre du procédé décrit ci-dessus, les composés de formule IV dans laquelle $R^1$, $R^2$, $R^3$, $R^4$, $R^5$ et X sont définis comme précédemment.

Les composés de formule I, ainsi que leurs sels d'addition, présentent une stabilité satisfaisante. Il possèdent des propriétés pharmacologiques intéressantes, et notamment une inhibition de l'activité enzymatique de l'aldéhyde réductase.

On a découvert en outre que ces composés sont capables d'agir sélectivement sur les cellules transformées en bloquant la division cellulaire, et donc d'inhiber sélectivement la croissance desdites cellules transformées, alors que l'inhibition de la croissance des cellules normales est nulle ou très réduite.

Ces propriétés, jointes à une toxicité réduite, permettent d'employer les composés de formule I, ou leurs sels d'addition, notamment dans le traitement des cancers, comme agents anti-tumoraux.

En particulier, les composés de formule I sont utilisables dans le traitement des formes de cancer, par exemple certaines leucémies, caractérisées par une augmentation de l'activité d'aldéhyde réductase dans les cellules tumorales, par rapport aux cellules normales ; voir notamment R. FELSTED et Coll., "Human Liver Daunorubicin - Reductases", in Progress in Clinical and Biological Research, Vol. 114, Ed. Alan R. Liss Incorp., 1982, pp.291-305.

L'invention concerne en particulier des compositions pharmaceutiques comprenant comme ingrédient actif au moins un composé de formule I ou un sel d'addition correspondant.

Ces compositions pharmaceutiques comprennent en outre un excipient permettant l'administration du médicament par voie orale, intratumorale, intramusculaire ou intraveineuse, ou encore sous forme d'implant.

La posologie varie avec la maladie traitée, le mode d'administration et l'état du malade. Généralement la dose quotidienne de composé de formule I varie de 10 à 500 mg/kg de poids corporel.

L'invention a également pour objet l'association des composés de ... d'autres agents antitumoraux. L'invention concerne, en

particulier, l'association des composés de formule I avec des composés de formule :

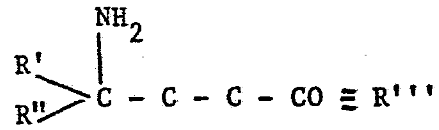

(V)

dans laquelle R' et R" représentent chacun un groupement hydrocarboné éventuellement substitué et/ou pouvant comporter des hétéroatomes, ou bien R' et R" représentent ensemble, un groupement hydrocarboné éventuellement substitué formant, avec l'atome d'azote auquel ils sont attachés, un cycle pouvant comporter des hétéroatomes, et R''' représente -H, un groupement alkyle inférieur ou un groupement $NH_2$ ; ainsi qu'avec les dérivés fonctionnels du groupement carbonyle et les sels d'addition de ces composés de formule V. Parmi les composés de formule V, on peut citer notamment :

- ceux pour lesquels R' et R" représentent chacun un groupement alkyle ayant 1 à 5 atomes de carbone ;
- ceux pour lesquels R' et R" représentent un groupement alkylène ayant 4 ou 5 atomes de carbone dans la chaîne ;
- ceux pour lesquels R''' représente H ou un groupement alkyle ayant 1 à 5 atomes de carbone.

Parmi les dérivés fonctionnels du groupement carbonyle des composés de formule V, on citera notamment les dialkyl cétals.

Les composés de formule V, ainsi que leurs dérivés et sels d'addition sont décrits dans la demande de brevet français n°83.12863 déposée le 4 Août 1983 et intitulée "Nouveaux dérivés acétyléniques possédant une activité d'inhibiteurs d'enzymes, leur préparation et leur application comme médicament".

Ces composés de formule V sont des agents anti-tumoraux qui présentent des propriétés pharmacologiques différentes de celles des composés de la présente demande. En particulier les composés de formule V sont des inhibiteurs de l'aldéhyde déshydrogénase.

On peut encore associer avantageusement, pour le traitement des tumeurs, les composés de formule I avec d'autres agents anti-tumoraux connus tels que la daunorubicine ou l'adriamycine.

L'invention a également pour objet l'utilisation des composés de formule I, ainsi que de leurs sels d'addition, dans la préparation industrielle de médicaments anti-tumoraux.

L'invention concerne également l'utilisation des composés de formule I dans la détection de l'état cancéreux ou pré-cancéreux de cellules d'une biopsie.

On a en effet observé que l'activité d'aldéhyde réductase est augmentée chez les cellules malignes mais aussi, à un stade précoce chez les

cellules transformées bénignes pouvant caractériser un état pré-cancéreux. Un test d'inhibition de la croissance cellulaire à l'aide d'un dérivé de formule I permet donc un diagnostic précoce.

En outre, le diagnostic peut être précisé en utilisant conjointement un test d'inhibition de l'activité d'aldéhyde déshydrogénase à l'aide d'un composé de formule V. En effet, l'activité d'aldéhyde déshydrogénase est modifiée chez les cellules malignes, mais non chez les cellules transformées bénignes.

Le test permettant de caractériser ces modifications de l'activité cellulaire peut être par exemple un test de croissance cellulaire en présence d'un composé de formule I, et un test de croissance cellulaire en présence d'un composé de formule V. Si les composés de formules I et V inhibent tous deux la croissance cellulaire, cela caractérise un état cancéreux alors que l'inhibition par les composés de formule I seuls, caractérise un état pré-cancéreux.

Les exemples suivants illustrent l'invention sans toutefois la limiter.

EXEMPLE 1

Préparation de l'époxyde de formule I, dans laquelle $R^1=R^2=R^3=CH_3$ et $R^4=R^5=-H$.

a) A une solution de 2,08 g (0,025 mole) d'amine propargylique dans 100 ml de THF anhydre, refroidie à -60°C, on ajoute sous azote 18,5 ml (0,03 mole) d'une solution 1,65M de n-butyl lithium dans l'hexane.

On laisse la température remonter en 30 minutes à -5°C puis refroidit à nouveau à -60°C avant d'ajouter une solution de 2,20 g (0,024 mole) de chloro-1 propanone-2 dans 5 ml de THF anhydre. On laisse alors la température remonter à 20°C en 3 heures.

On dilue à l'éther éthylique puis hydrolyse par 10 ml d'une solution saturée de $NH_4Cl$. Après traitement habituel, on récupère 3,72 g de chloro-6-hydroxy-5 diméthyl-2,5 hexyne-3 amine-2 (Rdt 85%).
F = 38-40°C (éther).

Les spectres I.R. et R.M.N. confirment la structure indiquée

b) A une solution de 1,76 g (0,01 mole) de la chlorhydrine, obtenue à l'étape précédente, dans 50 ml d'éthyléther anhydre, refroidie à -20°C, on ajoute sous vigoureuse agitation 1,41 g (0,013 mole) de tertiobutylate de potassium.

On laisse revenir à 20°C en 30 minutes puis lave par 2 x 10 ml d'une solution saturée de NaCl. Après traitement habituel, on récupère 1,09 g d'époxy-5,6 diméthyl-2,5 hexyne-3 amine-2 ((Rdt : 78%)

IR : 3340, 3280, 3040, 2220, 1590, 1370, 1270 nm

RMN :

| | | |
|---|---|---|
| Groupement diméthyle | 1,25 s | (6H) |
| Groupement méthyle | 1,40 s | (3H) |
| Groupement $NH_2$ | 1,45 s | (2H) |
| Groupement méthylène | 2,50 d(J+6Hz) | (1H) |
| | 2,75 d | (1H) |

Masse $M^{\dagger}$ = 139(1), 138(2), 124(100), 108(15), 94(31), 82(14), 67(17), 58(9), 42(34).

EXEMPLE 2

ETUDE DES PROPRIETES PHARMACOLOGIQUES

a) Test in vitro

On prépare des homogénats cellulaires de cellules NRK B77. Des homogénats contenant environ 500 microgrammes de l'enzyme sont incubés avec le composé de l'exemple 1 aux doses de 0,2 mM, de 0,8 mM et de 1 mM. On laisse incuber pendant 15, 30 ou 60 minutes.

Après incubation, une aliquote est ajoutée au substrat, qui est ici le dialdéhyde malonique 2mM dissous dans un tampon phosphate de pH6 contenant 0,35 mM de NADPH (co-facteur de l'enzyme).

La densité optique est mesurée toutes les minutes, pendant 5 minutes, à 340 nm.

La transformation de NADPH en NADP provoque une diminution linéaire de la densité optique. On évalue l'effet inhibiteur par la différence entre la variation de la densité optique chez les témoins (sans inhibiteur) et la variation de la densité optique avec l'inhibiteur.

A la dose de 1 mM, le composé de l'exemple 1 provoque une inhibition totale de l'activité ALDR au bout de 30 minutes. Avec 0,2 mM, l'activité restante est inférieure à 25% au bout de 60 minutes. A la dose de 0,8 mM, l'activité ALDR restante au bout de 30 minutes est d'environ 10%.

b) Inhibition de la croissance de cellules transformées

Les expériences sont effectuées de la façon suivante :

On ensemence avec $2.10^5$ cellules étudiées un milieu de culture constitué par le milieu minimum Eagle additionné de 10% de sérum de veau.

Au bout de 4 heures après l'ensemencement, on ajoute le composé de formule I aux doses indiquées dans le tableau ci-après. Après 4 jours de culture, on mesure l'inhibition par rapport aux cultures témoins non traitées.

Les résultats sont indiqués dans le tableau I ci-après ;

c) Toxicité

L'étude de la toxicité a été effectuée sur des souris. On a administré le produit de l'exemple 1 par voie intrapéritonéale à la dose de 3 g/Kg par jour pendant 8 jours. Aucune toxicité apparente n'a été décelée.

La DL 50 est donc supérieure à 3 g/Kg.

TABLEAU I

EFFET DU PRODUIT DE L'EXEMPLE 1 SUR LA CROISSANCE CELLULAIRE
EXPRIME EN POURCENTAGE D'INHIBITION

| | $5.10^{-5}M$ | $1.10^{-4}M$ | $2.10^{-4}M$ | $4.10^{-4}M$ | $6.10^{-4}M$ |
|---|---|---|---|---|---|
| Cellules diploïdes normales | | | | | |
| . MRC5 | 0 | 0 | 0 | 0 | 0 |
| Lignées établies | | | | | |
| . NRK | - | - | 30 | 60 | Mort |
| Cellules transformées | | | | | |
| . HeLa | 0 | 0 | 20 | 80 | 90 |
| . HEp2 | 0 | 0 | 20 | 80 | Mort |
| . 293 | 20 | 50 | 90 | Mort | Mort |
| . NRKB77 | 0 | 0 | 0 | 50 | 80 |

NRK : Cellules de rein de rat normales

NRKB77 : Cellules de rein de rat transformées par le virus du Sarcome du Rous (Souche B77)

HeLa : Carcinome utérin humain

MRC5 : Fibroblastes normaux de poumon embryonnaire humain

HEp2 : Carcinome de larynx humain

293 : Rein d'embryon humain transformé par le DNA de l'adénovirus type 5.

EXEMPLES 3 à 5

De façon analogue à celle décrite à l'exemple 1, au départ des composés de formules II et III correspondants, on a préparé les composés de formule I suivants :

| Exemple | $R_1$ | $R_2$ | $R_3$ | $R_4$ | $R_5$ |
|---|---|---|---|---|---|
| 3 | $CH_3$ | $CH_3$ | $CH_3$ | $CH_3$ | H |
| 4 | $CH_3$ | $CH_3$ | $C_6H_5$ | H | H |
| 5 | $CH_3$ | $CH_3$ | H | H | H |

Ces composés possèdent notamment une activité inhibitrice de la croissance des cellules transformées.

EXEMPLES 6 et 7

De façon analogue, au départ des composés de formules II et III correspondants, on obtient les composés suivants :

| Exemple | $R_1$ | $R_2$ | $R_3$ | $R_4$ | $R_5$ |
|---|---|---|---|---|---|
| 6 | $-C_5H_{10}-$ | | $CH_3$ | H | H |
| 7 | $CH_3$ | $C_2H_5$ | $CH_3$ | H | H |

EXEMPLE 8 : Détection de cellules transformées dans une biopsie

Comme indiqué ci-dessus, les composés de formule I peuvent être utilisés pour détecter l'état cancéreux ou pré-cancéreux de cellules d'une biopsie.

L'invention a,en particulier,pour objet un procédé de détection de l'état cancéreux ou précancéreux de cellules d'une biopsie, caractérisé par le fait que l'on met en culturè des cellules de la biopsie, dans un milieu de culture approprié, respectivement en présence et en l'absence d'un composé de formule I, le composé de formule I étant introduit, lorsqu'il est présent, à une concentration à laquelle il inhibe la croissance de cellules transformées connues, concentration pouvant aller par exemple de ($10^{-5}$ M à $10^{-2}$M pour une culture contenant au moins $10^{5}$ cellules étudiées au moment de l'addition du composé de formule I), puis que l'on compare la croissance des cellules cultivées en présence du composé de formule I à la croissance des cellules en l'absence du composé de formule I, étant entendu qu'une inhibition significative, par exemple d'au moins 50 %,de la croissance des cellules, par rapport aux cellules des cultures non traitées, signifie que la biopsie contenait des cellules transformées (bénignes ou malignes).

Le test de détection précédent peut être complété par un test complémentaire, également objet de l'invention, permettant d'établir si les cellules transformées sont des cellules bénignes ou malignes.

L'invention a donc aussi pour objet un procédé de détection de cellules malignes éventuellement présentes dans une biopsie, caractérisé par le fait que l'on réalise des cultures des cellules de la biopsie dans un milieu de culture approprié, respectivement en présence et en l'absence d'un composé de formule V introduit, lorsqu'il est présent, à une concentration à laquelle le composé de formule V inhibe la croissance de cellules malignes connues, puis que l'on compare la croissance des cellules en présence du composé de formule V à la croissance des cellules en l'absence du composé de formule V, étant entendu qu'une inhibition significative par exemple d'au moins 50 % de la croissance des cellules, par rapport aux cellules des cultures non traitées, signifie que la biopsie contenait des cellules transformées malignes. Dans le cas où les cellules de départ avaient déjà été soumises au test décrit ci-dessus concernant l'utilisation d'un composé

de formule I, on peut non seulement déterminer si la biopsie contenait des cellules transformées, mais aussi si les cellules transformées sont des cellules bénignes ou des cellules malignes.

Le composé de formule V est utilisé par exemple à une concentration de $10^{-5}$ M à $10^{-2}$ M pour une culture contenant au moins $10^5$ cellules au moment de l'addition du composé de formule V.

En pratique, on réalise des essais avec des concentrations croissantes du produit de formule V.

Le test de détection des cellules transformées avec un composé de formule I peut être mis en oeuvre par exemple selon le procédé décrit ci-dessus à l'exemple 2 (b).

Le test de détection permettant de distinguer entre les cellules transformées bénignes et les cellules transformées malignes, à l'aide d'un composé de formule V, peut être mis en oeuvre par exemple de la façon suivante.

On ensemence des boîtes de Petri de diamètre 10 cm avec $10^6$ cellules étudiées. Au bout de 4 heures après l'ensemencement, on ajoute le composé de formule V (avec R' = R" = $CH_3$, R"' = H) sous la forme d'une solution aqueuse contenant NaCl 0,14 M et ayant un volume de 100 microlitres. L'effet du composé de formule V est étudié à diverses concentrations. On a obtenu les résultats suivants résumés dans le tableau II.

| | Concentrations utilisées | | | | |
|---|---|---|---|---|---|
| | $0,5.10^{-5}$ M | $10^{-4}$ M | $2.10^{-4}$ M | $4.10^{-4}$ M | $6.10^{-4}$ M |
| Cellules normales | | | | | |
| CEC | 0 | 0 | 0 | 10 | 12 |
| MRC5 | 0 | 0 | 0 | 0 | 18 |
| Lignées cellulaires établies (cellules transformées bénignes) | | | | | |
| NRK | 0 | 0 | 0 | 0 | 0 |
| Cellules malignes | | | | | |
| NRKB 77 | 0 | 16 | 31 | 57 | 79 |
| HeLa | 0 | 18 | 36 | 78 | 91 |

CEC signifie : culture de cellules normales d'embryon de poulet. Les cellules ne peuvent être cultivées qu'avec un nombre de passages limités (3 à 5).

MRC5 est une culture de cellules normales (fibroblaste de poumon d'embryon humain). Les cellules connues qui ne peuvent être cultivées qu'avec un nombre de passages limités (28 à 30), ont été fournies par l'INSTITUT MERIEUX LYON, FRANCE.

NRK (ATCC CRL 1571) : culture de cellules de rein de rat cultivables indéfiniment mais ne donnant pas naissance à des tumeurs lorsqu'elles sont injectées ; ce sont donc des cellules transformées bénignes.

NRK B 77 : ce sont des cellules de rein de rat transformées cultivables indéfiniment et donnant naissance à des tumeurs lesquelles sont injectables ; ce sont donc des cellules malignes. Les cellules NRK B 77 utilisées ont été fournies par le Dr. Thomas GRAF (HEIDELBERG).

Hela : il s'agit de la lignée cellulaire maligne humaine bien connue (ATCC CCL 2).

## REVENDICATIONS

1. Les composés de formule générale I :

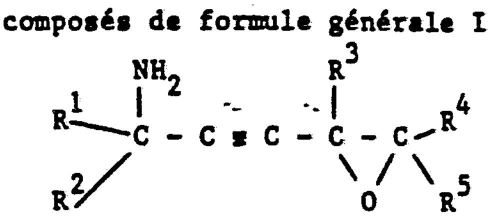

(I)

dans laquelle $R^1$ et $R^2$ représentent chacun indépendamment un groupement hydrocarboné pouvant être substitué et/ou comporter des hétéroatomes, ou bien $R^1$ et $R^2$ représentent ensemble un groupement hydrocarboné éventuellement substitué formant, avec l'atome de carbone auxquels ils sont attachés, un cycle pouvant comporter des hétéroatomes,

$R^3$ représente -H ou un groupement alkyle inférieur ou aryle,

et $R^4$ et $R^5$ représentent chacun indépendamment, -H ou un groupement hydrocarboné ; ainsi que leurs sels d'addition.

2. Composés selon la revendication 1, caractérisés par le fait que $R^1$ et $R^2$ représentent chacun indépendamment un groupement alkyle ayant 1 à 5 atomes de carbone, ou $R^1$ et $R^2$ représentent ensemble un groupement pentaméthylène.

3. Composés selon l'une quelconque des revendications 1 et 2, caractérisés par le fait que $R_3$ représente un groupement alkyle ayant 1 à 5 atomes de carbone ou un groupement phényle.

4. Composés selon l'une quelconque des revendications précédentes, caractérisés par le fait que $R_4$ et $R_5$ représentent chacun -H, ou l'un des substituants $R_4$, $R_5$ représente -H et l'autre représente un alkyle ayant 1 à 5 atomes de carbone.

5. Procédé de préparation d'un composé selon l'une quelconque des revendications précédentes, caractérisé par le fait que l'on utilise comme produit de départ un composé de formule II :

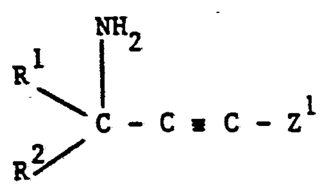

(II)

dans laquelle $Z^1$ représente soit -H soit un équivalent d'un métal ou d'un groupement lié à l'atome de carbone auquel il est attaché par un liaison carbone-métal, que l'on fait réagir ledit composé de départ avec un dérivé carbonylé halogéné de formule III :

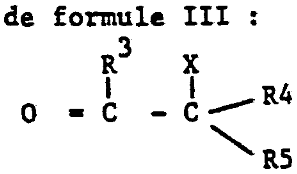

(III)

dans laquelle $R^3$, $R^4$ et $R^5$ sont définis comme précédemment et X représente un

atome d'halogène, pour obtenir une halohydrine de formule IV :

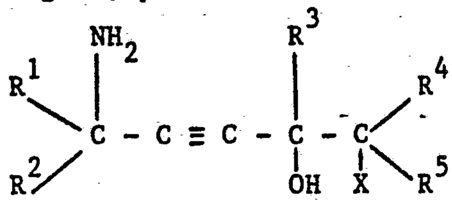

(IV)

dans laquelle $R^1$, $R^2$, $R^3$, $R^4$, $R^5$, $Z^1$ et X sont définis comme précédemment, et que l'on soumet ledit composé de formule IV à l'action d'une base pour obtenir un composé de formule I, puis que l'on soumet le cas échéant le composé obtenu à une réaction de salification du groupement amine.

6. Compositions pharmaceutiques caractérisées par le fait qu'elles comprennent comme ingrédient actif au moins un composé de formule I, ou un sel d'addition correspondant, tel que défini dans l'une quelconque des revendications 1 à 4.

7. Compositions pharmaceutiques, caractérisées par le fait qu'elles comprennent, en association, au moins un composé de formule I tel que défini dans l'une quelconque des revendications 1 à 4, ou un de ses sels d'addition, avec au moins un autre agent anti-tumoral.

8. Compositions pharmaceutiques selon la revendication 7, caractérisées par le fait que ledit autre agent anti-tumoral est la daunorubicine ou l'adriamycine.

9. Compositions pharmaceutiques selon la revendication 7, caractérisées par le fait que ledit autre agent anti-tumoral est soit un composé de formule V :

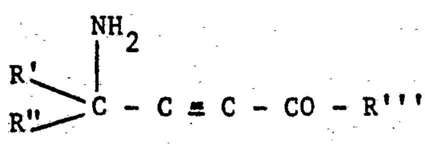

(V)

dans laquelle R' et R" représentent chacun un groupement hydrocarboné éventuellement substitué et/ou pouvant comporter des hétéroatomes, ou bien R' et R" représentent ensemble, un groupement hydrocarboné éventuellement substitué formant, avec l'atome d'azote auquel ils sont attachés, un cycle pouvant comporter des hétéroatomes, et R''' représente -H, un groupement alkyle inférieur ou un groupement $NH_2$ ; soit un dérivé fonctionnel du groupement carbonyle ou un sel d'addition d'un composé de formule V.

10. Utilisation d'un composé de formule I tel que défini dans l'une quelconque des revendications 1 à 4, ou de ses sels d'addition, dans la préparation industrielle de médicaments anti-tumoraux ou dans la détection d'états cancéreux ou pré-cancéreux sur des cellules de biopsie.

11. A titre de produits intermédiaires obtenus dans le procédé de la revendication 5, les composés de formule IV :

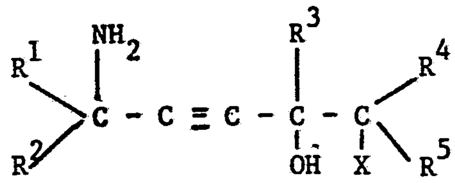

(IV)

dans laquelle $R^1$, $R^2$, $R^3$, $R^4$, $R^5$ et X sont définis comme dans la revendication 5.

12. Procédé de détection de l'état cancéreux ou pré-cancéreux de cellules d'une biopsie, caractérisé par le fait que l'on met en culture des cellules de la biopsie, dans un milieu de culture approprié, respectivement en présence et en l'absence d'un composé de formule I, le composé de formule I étant introduit, lorsqu'il est présent, à une concentration à laquelle il inhibe la croissance de cellules transformées connues, puis que l'on compare la croissance des cellules cultivées en présence du composé de formule I à la croissance des cellules en l'absence du composé de formule I, étant entendu qu'une inhibition significative, par exemple d'au moins 50 % de la croissance des cellules, par rapport aux cellules des cultures non traitées, signifie que la biopsie contenait des cellules transformées (bénignes ou malignes).

13. Procédé de détection de cellules malignes éventuellement présentes dans une biopsie, caractérisé par le fait que l'on réalise des cultures des cellules de la biopsie dans un milieu de culture approprié, respectivement en présence et en l'absence d'un composé de formule V introduit, lorsqu'il est présent, à une concentration à laquelle le composé de formule V inhibe la croissance de cellules malignes connues, puis que l'on compare la croissance des cellules en présence du composé de formule V à la croissance des cellules en l'absence du composé de formule V, étant entendu qu'une inhibition significative par exemple d'au moins 50 % de la croissance des cellules, par rapport aux cellules des cultures non traitées, signifie que la biopsie contenait des cellules transformées malignes.

Office européen des brevets

**RAPPORT DE RECHERCHE EUROPEENNE**

0193432

Numero de la demande

EP 86 40 0207

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| A | EP-A-0 130 829 (ROOPEROL)<br>* En entier *<br>----- | 1-13 | C 07 D 303/36<br>A 61 K 31/335<br>C 07 C 91/15 |
| | | | **DOMAINES TECHNIQUES RECHERCHES (Int. Cl.4)** |
| | | | C 07 D 303/00<br>C 07 C 91/00 |

Le présent rapport de recherche a été établi pour toutes les revendications

| Lieu de la recherche | Date d'achèvement de la recherche | Examinateur |
|---|---|---|
| LA HAYE | 09-05-1986 | ALLARD M.S. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503 03 82